# EUROPEAN PATENT APPLICATION

(11) **EP 4 714 485 A1**
(43) Date of publication of application: **25.03.2026**
(21) Application number: 25198077.7
(22) Date of filing: 26.08.2025
(51) Int. Cl.: A61M 25/00, A61M 39/10, A61M 25/01

(54) **LEFT ATRIAL APPENDAGE OCCLUDER DELIVERY SYSTEM**

(30) Priority: 18.09.2024 US 202463695909 P
(71) Applicant: St. Jude Medical, Cardiology Division, Inc., St. Paul, MN 55117 (US)
(72) Inventor: PERSZYK, Brian Joseph, Shoreview, 55126 (US); BRAGG, Ellie, St. Louis Park, 55416 (US)
(74) Representative: Gill Jennings & Every LLP

(57) **Abstract**

A delivery device includes a catheter sheath extending distally from the handle, a sheath hub coupled to the catheter sheath, the sheath hub having a body including at least one proximal aligning feature, a dilator insertable within the catheter sheath, and a dilator hub coupled to the dilator, the dilator hub having a hub aligning feature configured to couple to the at least one proximal aligning feature of the sheath hub.

## Description

### Cross-Reference to Related Applications

This application claims the benefit of U.S. Provisional Patent Application No. 63/695,909, filed on September 18, 2024, the entire contents of which are hereby incorporated by reference in their entirety.

### Background

Catheters are frequently used to assist in the delivery of medical devices into a patient non-invasively. For example, several types of collapsible and expandable medical devices may be delivered to, and implanted within, the heart of a patient using a catheter that is advanced through the vasculature and into the patient's heart without needing to make any incisions in the patient's chest or heart, and without needing to put the patient on cardiopulmonary bypass.

Left atrial appendage ("LAA") occluder devices are one example of collapsible and expandable medical devices that may be delivered to a patient's heart via a catheter that traverses the patient's vasculature. In some examples, a catheter may be advanced through the patient's femoral vein, into the right atrium through the inferior vena cava, across the atrial septum and into the left atrium, with a distal end of the catheter positioned within or adjacent to the LAA. The LAA occluder device may be within the catheter during the advancement of the catheter, or otherwise may be advanced through the catheter after the catheter is already in the desired position. The LAA occluder may be in a collapsed state with a relatively small profile while inside the catheter, and may self-expand into the LAA upon deployment from the distal end of the catheter. One such LAA occluder is the Amplatzer^{™} Amulet^{™} Occluder offered by Abbott Labs. One example of a LAA occluder device is described in U.S. Patent No. 10,201,337, the disclosure of which is hereby incorporated by reference herein.

As explained in greater detail below, although this disclosure generally focuses on a sheath hub and dilator hub in the context of a steerable sheath for delivering a LAA occluder, the disclosure is not so limited, and may apply to various other types of sheaths (including non-steerable sheaths) and various other types of medical devices to be delivered (including other occluder-type devices, such as PFO closure devices, and other devices that are not occluders).

### Brief Summary

In some embodiments, a delivery device includes a catheter sheath extending distally from a handle, a sheath hub coupled to the catheter sheath, the sheath hub having a body including at least one proximal aligning feature, a dilator insertable within the catheter sheath, and a dilator hub coupled to the dilator, the dilator hub having a hub aligning feature configured to couple to the at least one proximal aligning feature of the sheath hub.

In some examples, a delivery device includes a catheter sheath extending distally from a handle, a sheath hub coupled to the catheter sheath, the sheath hub having a body including at least one proximal aligning feature, a dilator insertable within the catheter sheath, and a dilator hub couplable to the sheath hub, the dilator hub having a rotation indicator configured to be circumferentially aligned with the proximal aligning feature of the sheath hub when the dilator hub is coupled to the sheath hub.

### Brief Description of the Drawings

Fig. 1A is a schematic view of a steerable sheath according to one aspect of the disclosure.
Fig. 1B is a schematic perspective view of a split pull ring according to one aspect of the disclosure.
Fig. 2A is a schematic view of a dilator for use with the steerable sheath of Fig. 1A.
Fig. 2B is a schematic view of another example of a dilator for use with the steerable sheath of Fig. 1A.
Fig. 3A is a schematic cross-sectional view of a sheath hub coupled to an interior mounting shaft of a handle according to the present disclosure.
Fig. 3B is a perspective view of the sheath hub of Fig. 3A.
Fig. 4A is a schematic cross-section of the mounting shaft of the handle according to one embodiment.
Fig. 4B is a schematic cross-section of the keyed feature of a sheath hub within the mounting shaft of Fig. 4A.
Fig. 4C is a schematic side view of the sheath hub of Fig. 3A.
Fig. 4D is a schematic cross-sectional view of a mounting shaft of a handle according to the present disclosure.
Fig. 4E is a schematic side view of a sheath hub being coupled to a dilator hub according to one embodiment.
Fig. 4F is a schematic perspective view of a sheath hub being coupled to a dilator hub according to another embodiment.
Fig. 4G is a schematic perspective view of a sheath hub being coupled to a dilator hub according to another embodiment.
Fig. 4H is a schematic perspective view of a dilator hub according to another embodiment.
Fig. 5A is a perspective view of an occluder in an expanded or deployed condition.
Fig. 5B is a schematic view of the occluder of Fig. 5A in a collapsed or delivery condition.
Fig. 5C is a side view of the occluder of Fig. 5A coupled to a delivery cable.

### Detailed Description

As used herein, the term proximal refers to a position relatively close to a user of a medical device, while the term distal refers to a position relatively far from the user of the medical device, when the medical device is being used in an intended manner. In other words, the leading end of a medical device is positioned distal to the trailing end of the medical device.

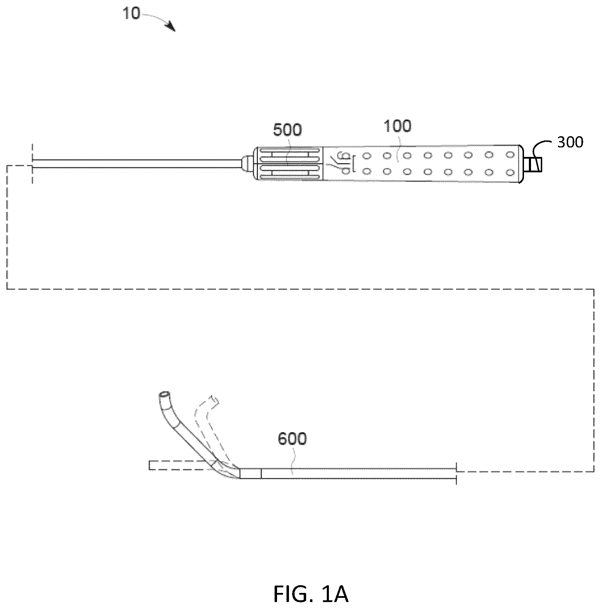

Fig. 1A illustrates a delivery device 10, which in the illustrated embodiment is a steerable sheath, although it should be understood that the inventive concepts disclosed herein may be used in conjunction with other catheter or sheath devices, whether or not steerable. Generally, delivery device 10 includes a handle 100, a sheath hub 300 at a proximal end of the handle 100, a deflection knob 500 at a distal end of the handle 100, and a deflectable sheath 600 extending from a distal end of the deflection knob 500 to a terminal distal end of the delivery device 10. This disclosure focuses on the sheath hub 300 and associated dilator hub, and the remainder of the delivery device 10 is generally described for the purpose of providing a contextual example of the use of sheath hub 300. It should be understood that sheath hub 300 described herein (including the optional variations described therewith) may be applicable to any suitable delivery device. Delivery device 10 is particularly suited for delivery of a collapsible and expandable LAA occluder, but it should be understood that the delivery device 10 may be suited to delivery of other medical devices.

The handle 100 may be a generally cylindrical or otherwise shaped member that the user of the delivery device 10 may grip during use. The handle 100 may be at least partially hollow and house various components therein, and may have one or more internal lumens so that medical devices may be passed through the delivery device 10 from the proximal end to and beyond the terminal distal end of the delivery device 10. The handle 100 may be rotatably coupled to the deflection knob 500, with the deflection knob 500 being rotatable about the central longitudinal axis of the handle 100. The deflection knob 500 may be operably coupled to two pull wires that traverse the deflectable sheath 600 and which are fixed to anchors (or pull rings or similar structures) near the distal tip of the sheath 600. Rotation of the deflection knob 500 in a first rotational direction may deflect the distal tip of the sheath 600 in a first deflection direction, while rotation of the deflection knob 500 in a second opposite rotational direction may deflect the distal tip of the sheath 600 in a second deflection direction opposite the first deflection direction. In one exemplary embodiment, the distal tip of the sheath may have a neutral angled position of about 45 degrees relative to the central longitudinal axis of the delivery device 10, with a maximum deflection (upon rotation of the deflection knob 500 in the first, e.g. clockwise, rotational direction) of about 120 degrees (shown in phantom lines in Fig. 1A) relative to the central longitudinal axis of the delivery device 10, and a minimum deflection (upon rotation of the deflection knob 500 in the second, e.g. counterclockwise, rotational direction) of about 0 degrees (shown in phantom lines in Fig. 1A) relative to the central longitudinal axis of the delivery device 10. In one example, the proximal end of each pull wire may be coupled to an axially slideable component within handle 100, where rotation of the deflection knob 500 causes the two axially slideable components to slide axially in opposite directions. Suitable pull wire mechanisms are described in greater detail in U.S. Patent No. 7,691,095, the disclosure of which is hereby incorporated by reference herein. The deflection mechanisms and ranges described above are merely exemplary, and as noted above, steering or deflection control may in some embodiments be entirely omitted from delivery device 10.

The catheter 600 may define a lumen therethrough configured to allow other devices to pass through the lumen. The catheter 600 may be formed from any suitable materials and in any suitable configuration. In one example, the catheter 600 includes an innermost liner layer, a torque transfer layer surrounding at least portions of the inner layer, and an outer sheath formed over the torque transfer layer. The wall of the catheter 600 may define lumens as well, for example two lumens spaced about 180 degrees apart, to accommodate the pull wires therethrough. Examples of suitable methods and materials for use in forming the catheter 600 are described in greater detail in U.S. Patent No. 7,914,515, the disclosure of which is hereby incorporated by reference herein. However, it should be understood that sheath hub 300 may be used with any suitable catheter configuration.

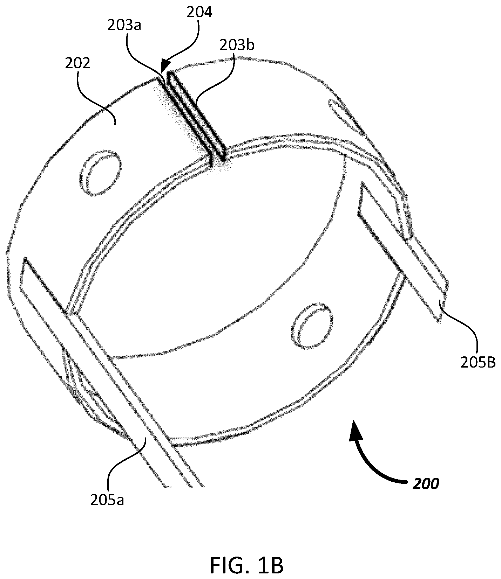

As noted, pull rings may be used to aid in steering the sheath 600. Examples of these pull rings and the associated steering is found in U.S. Serial No. 18/397,566, filed December 27, 2023, the disclosure of which is hereby incorporated by reference herein. One example of a split pull ring 200 is shown in Fig. 1B. In this example, sheath 600 may include a proximal pull ring and a distal pull ring, and may include a slit pull ring 200 as the proximal pull ring, the distal pull ring or both. As shown, split pull ring 200 may include a body 202 having a longitudinal split 204 that traverses the body and leaves a gap between two opposing edges 203a, 203b. Body 202 may be connected to two pull wires 205a,205b disposed on contralateral sides (e.g., 180 degrees from one another). Split pull ring 200 may allow the pull ring inner diameter to be sized approximately to the same outer diameter as the PTFE liner of sheath 600 (or slightly smaller), while still being able to advance it over the top of the sheath 600 without damaging the liner or liner lumens, as it's able to flex open during advancement over the liner. In some examples, the split pull ring 200 has an inner diameter of between 0.189 and 0.194 inches and the sheath 600 has an outer diameter of approximately 0.238 inches. In some examples, the split pull ring 200 has an outer diameter of 0.203 inches. It is hypothesized that using the smallest inner and outer diameter pull ring for either the proximal pull ring and/or the distal pull ring minimizes the material in the area of said pull ring, and therefore allows for one section of braid over the main portion of the shaft up to the distal pull ring while minimizing the larger diameter "bump" at the pull ring. This feature may also improve the ease of manufacturing and allow the outer extrusion to reflow more equally over the braid and pull ring. This may be particularly advantageous at the proximal pull ring. A split pull ring as shown may also allow a slightly thicker pull ring and pull wires, to aid in pull wire tensile strength, without affecting the sheath outer diameter. Due to differences in PPI and braid diameter, along with facilitating pull wire exit location for manufacturability, a second larger diameter section of braid may be utilized for the larger diameter proximal end of the shaft. In some examples, the shaft may contain one section of braid over the proximal portion of the shaft, and a second section of braid over the main portion of the shaft up to the distal pull ring. The sheath curvature radii may be increased in both proximal and distal curves to ease the path of the device as it travels through the sheath, and therefore, reduce occluder use forces.

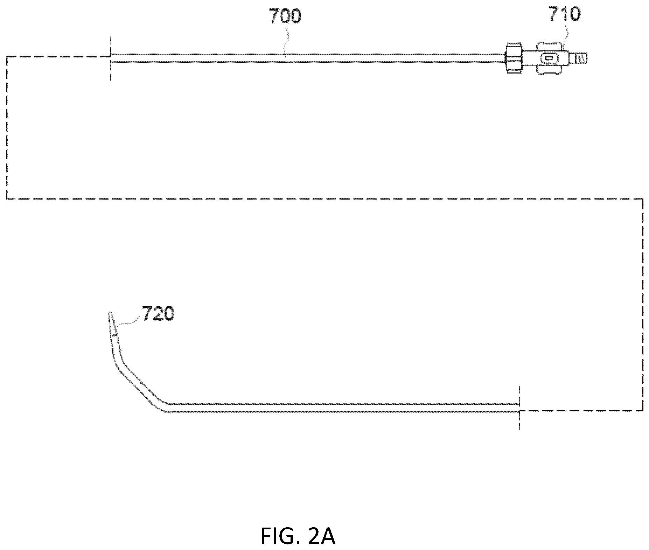

Fig. 2A illustrates a dilator 700 that may be used with delivery device 10. In the illustrated example, dilator 700 is a solid member that includes a connector 710 such as a luer lock at a proximal end thereof, and an atraumatic tip 720 at a distal end thereof. Although referred to as a "solid member," it should be understood that dilator 700 may include a guidewire lumen passing therethrough to allow for the dilator 700 to ride over a guidewire. In other words, dilator 700 may also be thought of as a "substantially solid" or thick-walled device. The distal end portion of the dilator 700 may, in the absence of applied forces, have an angle of about 45 degrees relative to the central longitudinal axis of the dilator 700. In other words, the dilator 700 may include a distal portion that has a neutral angle that is about the same as the neutral angle of the distal tip of the catheter 600, whether that angle is about 45 degrees or another value. The dilator 700 may have an outer diameter that is about equal to (or slightly smaller than) the inner diameter of the catheter 600. As will be described in greater detail below, the dilator 700 may be positioned within and through the catheter 600 during delivery of the delivery device 10 to the desired anatomical location. Then the dilator 700 may be removed to allow for other devices, such as an LAA occluder, to be passed into and through the delivery device 10 for implantation. In some embodiments, the distal portion of the dilator 700 may have curvature, or multiple angles generally similar to that shown in Fig. 2A. In an alternate embodiment to the dilator 700 shown in Fig. 2A, in other embodiments, the dilator may be completely straight (e.g., without any angles or curvature), or the distal portion may have an entirely straight portion that is at an angle relative to the main body of the dilator 700 (*e.g.,* a single angled portion instead of a multi-angled portion as shown in Fig. 2A). Dilator 700 may also be shapeable to facilitate transseptal puncture.

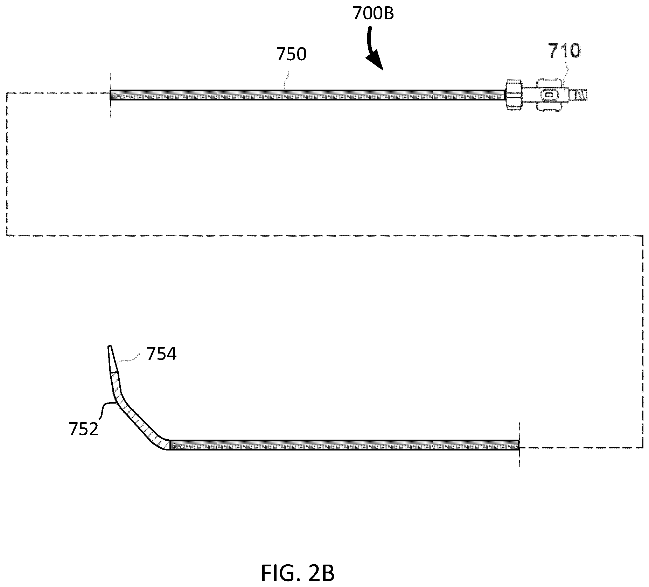

In some variations, shown in Fig. 2B, dilator 700B may be formed of multiple sections having different materials. For example, dilator 700B may include a combination of formable material(s) and deflectable material(s) disposed in certain sections to aid in transseptal puncture. In one example, a dilator 700 comprises a formable section 750 that includes a high-density polyethylene (HDPE) material which holds shape with moderate resistance to straightening. Alternatively, dilator 700 may comprise a formable section 750 that includes a full high-density polyethylene (HDPE) material extrusion or a more flexible extrusion (e.g. 65D pellethane, 45D-55D pebax, LDPE, or MDPE) with a metallic or polymeric hypo tube (e.g., 304SS) integrated for an increased resistance to straightening. In some examples, the formable section may comprise the majority of the extrusion length of dilator 700B. A deflectable section 752 may form the distal 1.5-3 inches of the dilator and may include the softer, deflectable material (e.g. 65D pellethane, 45D-55D pebax, LDPE, or MDPE). For example, the formable section 750 may comprise 65D pellethane with integrated hypotube and the deflectable section 752 may comprise 65D pellethane. This combination of materials allows the proximal shaft curve to be formed by the shapeable, stiffer portion of the dilator in the formable section 750 and the distal shaft to be articulated by the handle in the deflectable, softer deflectable section 752 of the dilator 700B. Optionally, dilator 700B may also have a third stiffer material at the tip section 754 that forms the final 0.75 inches-1.25 inches of the dilator 700B. The stiffer tip could be made from a 65D or stiffer Pellethane, 50D or stiffer PEBAX, or a HDPE/LDPE material, and may facilitate easier insertion into the groin and across the septum.

In some examples, a fully formable material may be used for the dilator. This dilator option may include a dilator formed of HDPE or HDPE or softer extrusion combined with a hypo tube, as previously discussed. This particular combination may rely on the shapeability (or formability) of the dilator to curve the shaft to the necessary shape for transseptal puncture by the physician prior to insertion into the body.

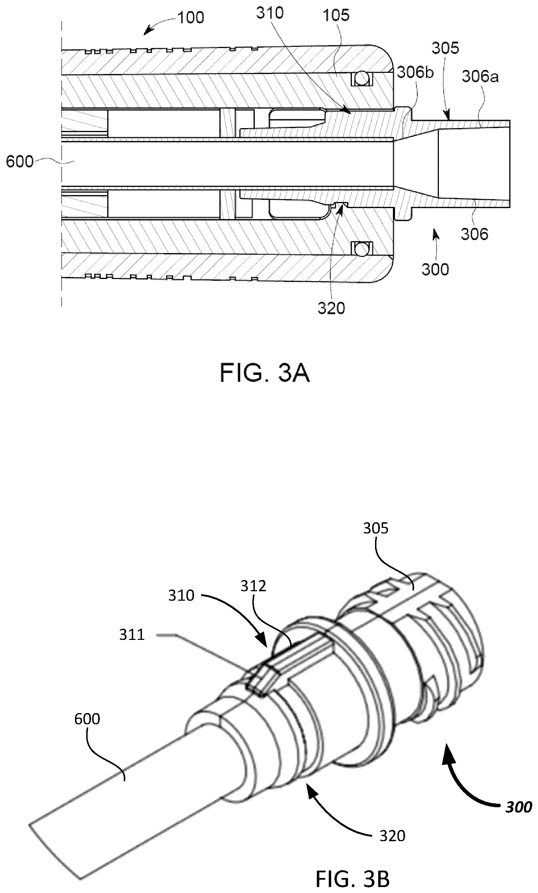

Fig. 3A shows a cross-sectional side view of a sheath hub 300 in an assembled condition with handle 100, and specifically with interior mounting shaft 105 of handle 100. In the assembled condition, sheath hub 300 directly abuts and is coupled to mounting shaft 105 of handle 100, and a proximal end of the catheter 600 is coupled to a distal end of the sheath hub 300. Sheath hub 300 may include a proximal body 305 defining a lumen 306. In some examples, body 305 is molded directly onto catheter 600. Body 305 may be injection molded and made from a suitable polymer (e.g., 63D PEBAX). Body 305 may have a luer-type connection on its proximal end for mating with other instruments (e.g., a loading assembly or other subsystem). In some examples, body 305, including both keyed features and snap features, may be unitarily formed. In some examples, lumen 306 has a tapered diameter that decreases from the proximal end of sheath hub 300 to the distal end. In the example shown, lumen 306 includes a multi-stage taper including a first taper 306a and a second taper 306b. Fig. 3A illustrates two additional features of sheath hub 300, each of which will be described in turn. First, sheath hub 300 may include a keyed feature 310 to prevent rotation with respect to handle 100. Second, sheath hub 300 may include a snap-feature 320 to prevent axial or longitudinal movement with respect to handle 100.

As best shown in the perspective view of Fig. 3B, sheath hub 300 may include an axially extending keyed feature 310 in the form of a generally rectangular projection that extends from the outer surface of body 305. In some examples, keyed feature 310 may include a ramp 311 with a gradually increasing height followed by a ridge 312 of constant height. As shown, a keyed feature 310 with a single projection is disposed on body 305, although it will be understood that multiple projections, disposed around the circumference, may be used. In the example shown, the keyed feature 310 includes a single projection disposed at the 12 o'clock position. In some examples, the keyed feature 310 may be disposed in the same plane as the pull wires that traverse the deflectable sheath 600 (e.g., the keyed feature 310, and two pull wires are all disposed within a single plane). Alternatively, keyed feature 310 may be disposed in a different plane than the pull wires that traverse the deflectable sheath 600.

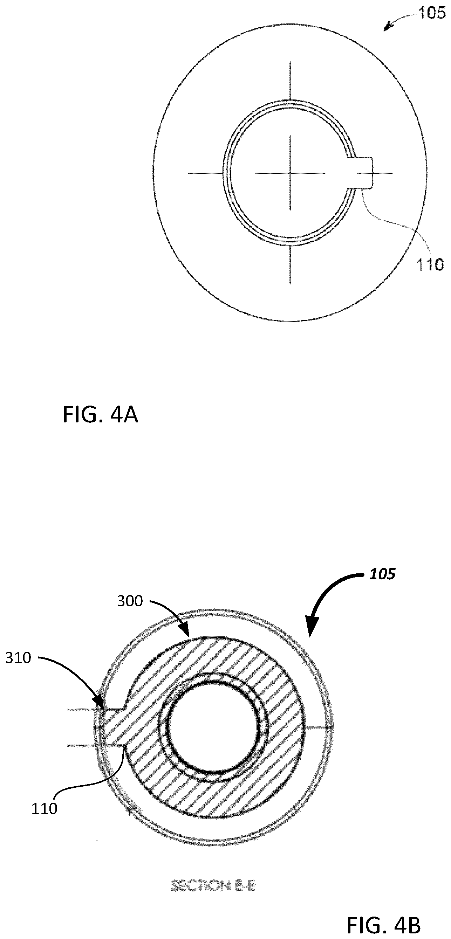

Turning to Figs. 4A-4B, it will be understood that mounting shaft 105 may include a slot 110 sized and spaced to receive keyed feature 310 of sheath hub 300. In some examples, slot 110 may have a same (or similar, or complementary) shape, size, height, and width of keyed feature 310. When keyed feature 310 of sheath hub 300 is properly engaged and/or mated with slot 110 of mounting shaft 105, the sheath hub 300 will be prevented from rotating relative to the mounting shaft 105 and handle 100. This feature may allow the user to safely and securely connect mating loaders, adapters, and/or dilators to the sheath hub 300 without the luer connection spinning in the handle.

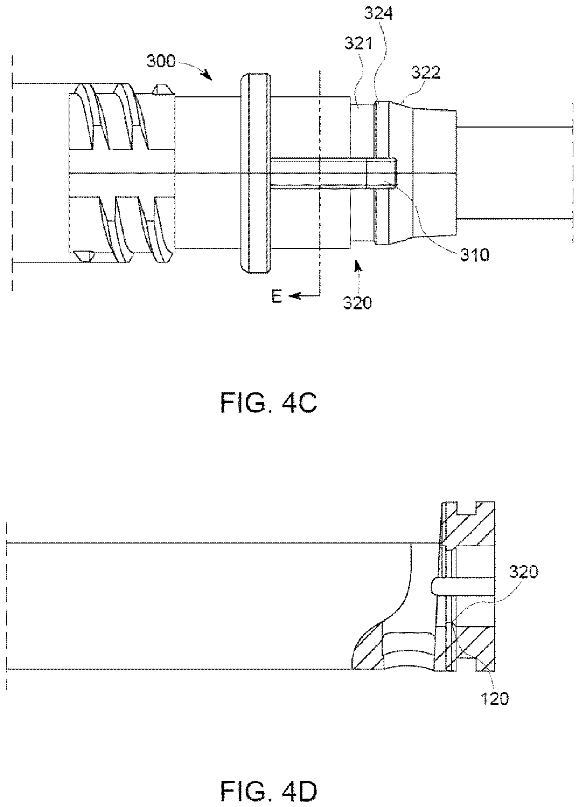

In addition to the keyed feature 310 to prevent rotation, a snap feature 320 (FIG. 4C) may prevent axial movement of the sheath hub 300 with respect to mounting shaft 105. Snap feature 320 may be formed in the shape of a circumferential or annular recess 321 formed in the outer surface of body 305. In some examples, body 305 includes a leading taper 322 that increases in diameter from the proximal end to the distal end, and then forms a ledge 324 with a constant diameter and a sudden drop-off adjacent recess 321. Recess 321 may be continuous or discontinuous. For example, recess 321 may be circumferentially disposed all around body 305 with the exception of the location at which keyed feature 310 is present. That is, the recess 321 may be interrupted by the keyed feature 310.

As shown in Fig. 4D, mounting shaft 105 may have a complementary rim 120 suitable for mating with snap feature 320. Specifically, in assembly, complementary rim 120 may ride onto leading taper 322, over ledge 324 and fall into recess 321 so that sheath hub 300 and mounting shaft 105 are irreversibly coupled and cannot be pulled apart without excessive force. Rim 120 may be continuous or discontinuous. For example, rim 120 may be circumferentially disposed all around mounting shaft 105 with the exception of the location at which keyed feature 310 of sheath hub 300 is present. That is, rim 120 may be interrupted by the keyed feature 310.

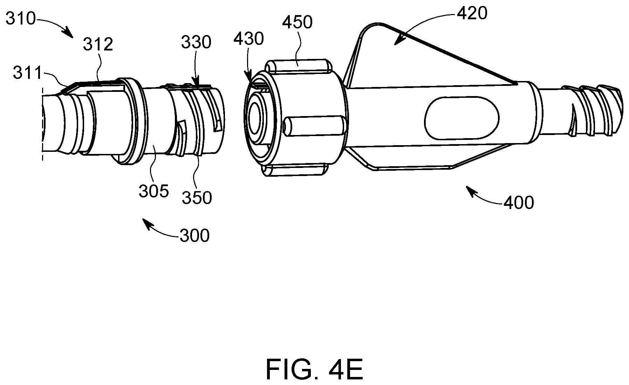

The dilator may also have complementary features to provide tactile feedback of curve direction when performing a procedure (e.g., transseptal puncture). As shown in Fig. 4E, a sheath hub 300 may include an axially extending keyed feature 310 in the form of a generally rectangular projection that extends from the outer surface of body 305. In some examples, keyed feature 310 may include a ramp 311 with a gradually increasing height followed by a ridge 312 of constant height. A proximal aligning feature 330 may be disposed on the sheath hub 300 opposite keyed feature 310. In some examples, proximal aligning feature 330 may include a flat surface, bevel or ridge capable of mating with a complementary feature on the dilator hub. Proximal aligning feature 330 may be circumferentially aligned with keyed feature 310 (e.g., both may be at the 12 o'clock position as shown in Fig. 4E). In this example, the dilator may have a dilator hub 400 that includes a rotation indicator 420 molded into the hub to provide tactile feedback of curve direction when performing a procedure (e.g., transseptal puncture). This allows the physician to ascertain the curve position relative to the patient while looking at imaging display. To align rotation indicator 420 with the curve direction, a hub aligning feature 430 may align, mate, and/or couple to proximal aligning feature 330 of sheath hub 300 to ensure that dilator curve indicator 420 rotationally matches the direction of the sheath curve. In this example, hub aligning feature 430 is formed as a flat surface that sits or rests on the proximal aligning feature 330. It will be understood that the shape of proximal aligning feature 330 and/or hub aligning feature 430 may be modified in various ways so long as the two features are complementary to one another. In some examples, dilator hub 400 will not mate or properly couple to sheath hub 300 without proximal aligning feature 330 and hub aligning feature 430 being properly positioned (e.g., rotationally aligned). Dilator hub 400 further includes a rotatable collar 450 configured to couple to external threads 350 of sheath hub 300. In some examples, rotatable collar 450 and hub aligning feature 430 are separately formed and collar 450 may be actuated independently of hub aligning feature 430.

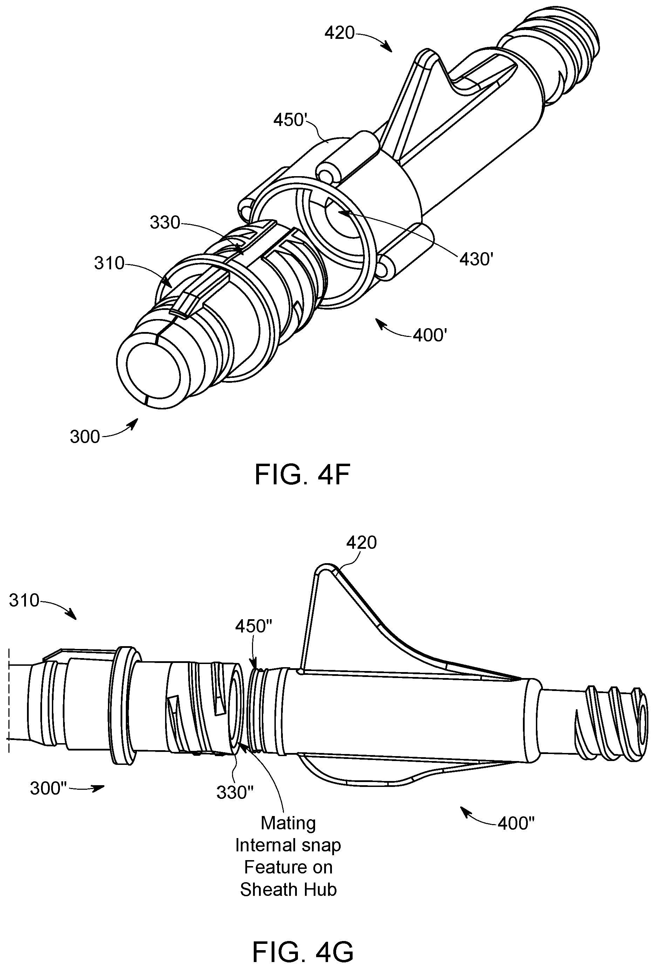

Fig. 4F shows an alternative embodiment in which a sheath hub 300 includes an axially extending keyed feature 310 that extends from the outer surface of body 305, and a proximal aligning feature 330. In this example, proximal aligning feature 330 is shown as a slotted depression or slit. The dilator may have a dilator hub 400' that includes a rotation indicator 420 molded into the hub to provide tactile feedback. Here, indicator 420 is configured to be fin-shaped, although other shapes of the indicator are possible. Dilator hub 400' may include a non-rotatable or fixed collar 450', and the collar may include an internally projecting hub aligning feature 430' configured to slide on or mate with proximal aligning feature 330. In this example, fixed collar 450' and hub aligning feature 430' are integrally formed, and the collar may be snap-fit with the sheath hub.

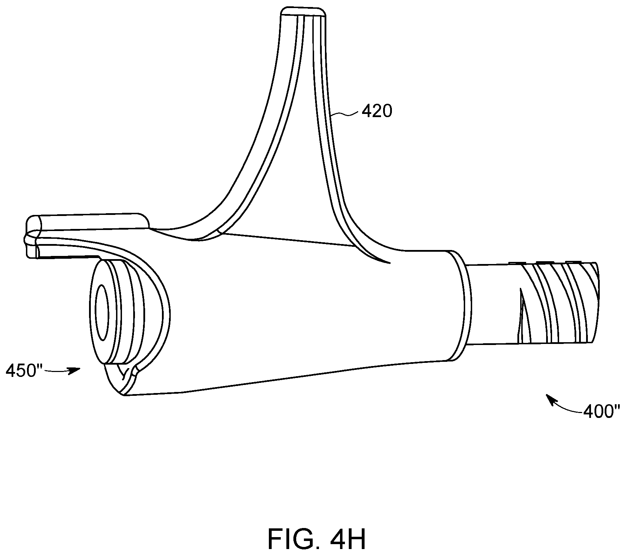

In yet another example, Fig. 4G shows a similar sheath hub 300" that includes an axially extending keyed feature 310 that extends from the outer surface of body 305. Sheath hub 300" may include an internal snap feature that functions as the proximal aligning feature 330", and the dilator may include a dilator hub 400" having a circumferential snap feature 450" that will couple to proximal aligning feature 330". Another view of a snap feature 450" is shown in Fig. 4H, where the snap feature is annular ring of reduced size for mating with a complementary ring of sheath hub 300". In this example, a collar is not shown, but it will be understood that a coupling rotatable or fixed collar, similar to those previously described, may be used.

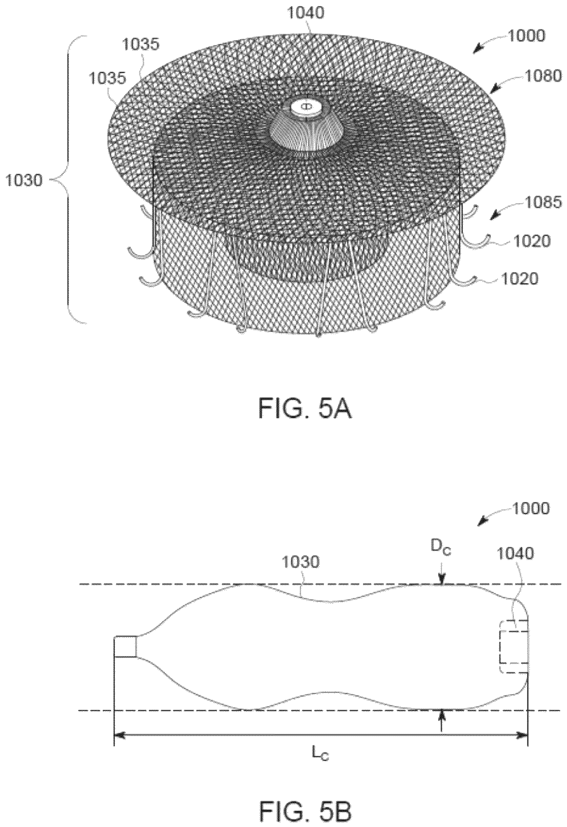

Fig. 5A is a perspective view of an occluder 1000, which may be a LAA occluder. Very generally, occluder 1000 includes an occluding material forming a tubular structure 1030, the occluding material being a braided metal fabric, which may be formed by braiding a plurality of strands 1035, which may be strands of nickel titanium alloy (*e.g.* under the tradename Nitinol), together and shape set (*e.g.* via heat setting) to the shape shown in Fig. 5A. When in the shape-set or expanded condition (*e*.*g*. in the absence of applied forces), the occluder 1000 may include a generally disk-shaped portion 1080 configured to abut an ostium of the patient's LAA, and a second generally cylindrical portion 1085 configured to be received within the patient's LAA. In some embodiments, the disk-shaped portion 1080 may be coupled to the cylindrical portion 1085 via a small diameter transition portion. The cylindrical portion 1085 may include one or more hooks 1020 configured to engage tissue within the LAA upon deployment. The disk-shaped portion 1080 may include a connector 1040, which may for example include internal threads, for coupling to a delivery cable.

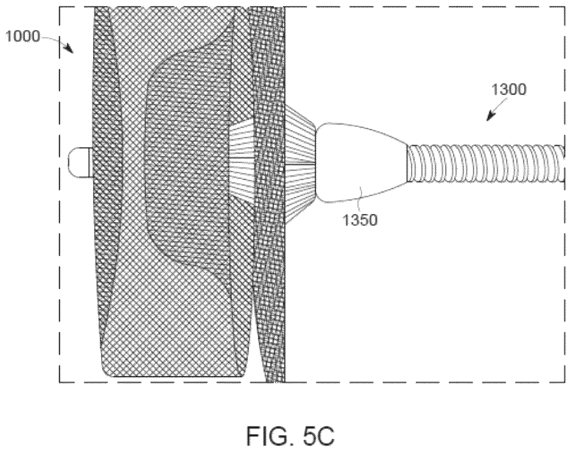

Fig. 5B illustrates occluder 1000 in a collapsed condition, having a collapsed diameter Dc smaller than the diameter of the disk-shaped portion 1080 and the cylindrical portion 1085 when in the expanded condition shown in Fig. 5A, and a collapsed length Lc longer than the length of the occluder in the expanded condition shown in Fig. 5A. Fig. 5C illustrates the delivery cable 1300 coupled to the connector 1040, for example via a threaded hub 1350 of the delivery cable 1300 that has been threaded into the connector 1040.

An exemplary use of the delivery device 10 for delivering occluder 1000 is described below. At the beginning of the procedure (or in preparation thereof), the delivery device 10, including dilator 700, may be removed from sterile packaging. The catheter 600 and the dilator 700 may be wiped with sterile gauze dampened with sterile saline to remove any foreign material that may be on the components. The user may then pass the dilator 700 through the delivery device 10 until the distal end of the dilator 700 passes the distal end of the catheter 600. When the dilator 700 is fully inserted through the delivery device 10, the connector 710 (or a threaded collar associated with the connector 710) may be rotated to couple to sheath hub 300, for example by inner threads of the connector 710 engaging outer threads of the sheath hub 300. Keyed feature(s) 310 and snap feature 320 of sheath hub 300 may prevent accidental detachment throughout the procedure.

The dilator 700 is now coupled to the remaining portions of the delivery device 10 as a single unit. Access may be gained to the patient via any suitable method, and a guidewire may be advanced into the patient's vasculature until it reaches the patient's left atrium, LAA, or pulmonary vein. Prior to or during introduction of the guidewire, a puncture may be made through the patient's atrial septum if the delivery route is, for example, through the patient's femoral vein and to the right atrium via the inferior vena cava, with the septal puncture allowing the guidewire and other components to traverse the atrial septum into the left atrium. With the guidewire in place, the dilator 700 (and the remainder of the delivery device 10 to which it is coupled) may be advanced over the guidewire into the patient until the distal end of the delivery device 10 reaches the left atrium or LAA. With the distal end of the delivery device 10 in the desired location, the connector 710 may be rotated to decouple it from sheath hub 300, and the dilator 700 may be withdrawn from the catheter 600, preferably slowly to prevent any ingress of air. After the dilator 700 is fully removed, the guidewire may next be fully removed from the delivery device 10 and the patient, although the guidewire may instead be simultaneously removed with the dilator 700.

With the distal end of the catheter 600 in the desired position, the occluder 1000 may be introduced into and through the delivery device 10 via a loading tube. Throughout the procedure, the keyed feature 310 and snap feature 320 may secure sheath hub 300 to mounting shaft 105 of handle 100 and prevent unintentional rotation or translation of the two components with respect to one another.

Although the sheath hub described herein may be particularly useful for the LAA occluder 1000 device described herein, it should be understood that this particular use is merely exemplary. For example, the sheath hub described herein may be particularly useful for other occluders formed of braided metal, including septal occluders, patent ductus arteriosus ("PDA") occluder devices, patent foramen ovale ("PFO") closure devices, *etc.* It should further be understood that the sheath hub described herein may work well with other occluders, including those not formed from a braided mesh, or any other medical device, whether an occluder or not and whether formed of braided mesh or not.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A delivery device comprising:
a catheter sheath extending distally from a handle;
a sheath hub coupled to the catheter sheath, the sheath hub having a body including at least one proximal aligning feature;
a dilator insertable within the catheter sheath; and
a dilator hub coupled to the dilator, the dilator hub having a hub aligning feature configured to couple to the at least one proximal aligning feature of the sheath hub.

2. The delivery device of claim 1, wherein the at least one proximal aligning feature includes at least one of a flat surface, a bevel or a ridge.

3. The delivery device of claim 1, wherein the sheath hub further comprises a keyed feature for preventing rotation of the sheath hub relative to a handle, the keyed feature and at least one proximal aligning feature being circumferentially aligned with one another.

4. The delivery device of claim 1, wherein the hub aligning feature of the dilator hub is configured to prevent rotation with respect to the proximal aligning feature of the sheath hub when the hub aligning feature and the proximal aligning feature are mated.

5. The delivery device of claim 1, wherein the dilator hub further includes a collar for coupling to a portion of the sheath hub.

6. The delivery device of claim 5, wherein the collar is configured to cover the hub aligning feature.

7. The delivery device of claim 1, wherein the catheter sheath includes at least one pull wire, and wherein the hub aligning feature is disposed in approximately a same plane as the at least one pull wire when the dilator hub is coupled to the sheath hub.

8. The delivery device of claim 1, wherein the catheter sheath includes at least one pull wire, and wherein the hub aligning feature is disposed in a different plane as the at least one pull wire when the dilator hub is coupled to the sheath hub.

9. The delivery device of claim 1, wherein the dilator hub further includes a rotation indicator spaced from the sheath hub aligning feature.

10. The delivery device of claim 9, wherein the dilator hub further includes a rotation indicator circumferentially aligned with the hub aligning feature.

11. The delivery device of claim 9, wherein the dilator hub further includes a rotation indicator configured to be circumferentially aligned with the proximal aligning feature when the dilator hub is coupled to the sheath hub.

12. The delivery device of claim 9, wherein the rotation indicator is fin-shaped.

13. The delivery device of claim 9, wherein the sheath hub and the dilator hub are configured to be snap fit with one another.

14. A delivery device comprising:
a catheter sheath extending distally from a handle;
a sheath hub coupled to the catheter sheath, the sheath hub having a body including at least one proximal aligning feature;
a dilator insertable within the catheter sheath; and
a dilator hub coupleable to the dilator, the dilator hub having a rotation indicator configured to be circumferentially aligned with the proximal aligning feature of the sheath hub when the dilator hub is coupled to the sheath hub.

15. The delivery device of claim 14, wherein the sheath hub and the dilator hub are configured to be snap fit with one another, or wherein the sheath hub and the dilator hub are configured to coupleable together via a collar.
